# EUROPEAN PATENT APPLICATION

(11) **EP 4 494 601 A1**
(43) Date of publication of application: **22.01.2025**
(21) Application number: 23770862.3
(22) Date of filing: 15.03.2023
(51) Int. Cl.: A61F 2/07, A61F 2/95, A61F 2/954

(54) **DEPLOYMENT DEVICE**

(30) Priority: 17.03.2022 JP 2022042519; 17.03.2022 JP 2022042679
(71) Applicant: SB-Kawasumi Laboratories, Inc., Kawasaki-shi, Kanagawa 210-8602 (JP); Sumitomo Bakelite Co., Ltd., Shinagawa-ku Tokyo 140-0002 (JP)
(72) Inventor: ISHIMARU, Yoshiki, Kawasaki-shi, Kanagawa 210-8602 (JP)
(74) Representative: Müller-Boré & Partner Patentanwälte PartG mbB
(86) International application number: PCT/JP2023/010190
(87) International publication number: WO 2023/176909

(57) **Abstract**

An indwelling device 1 for placing a tubular treatment device 10, which is radially expandable, in a bent site in a biological lumen includes: a sheath 20 configured to accommodate the tubular treatment device 10; and a long shaft-like member 30 configured to hold the tubular treatment device 10 and that to cause a distal side to advance and retreat inside the sheath 20 along an axial direction. The shaft-like member 30 includes a step portion 31a, 32a that protrudes in a radial direction of the shaft-like member 30, and a covering portion 33, 34 that covers a boundary portion between the step portion 31a, 32a and the shaft-like member 30 with an inclined surface 33a, 34a on a proximal side of the step portion 31a, 32a.

## Description

### Technical Field

The present invention relates to an indwelling device of a tubular treatment device.

### Background Art

In the related art, a tubular treatment device such as a stent graft, which is used for a treatment of an aneurysm or the like that occurs in a blood vessel wall, is known. Various proposals have been made for an indwelling device for delivering and placing a tubular treatment device in an affected area (refer to, for example, Patent Documents 1 and 2). In general, the indwelling device delivers the tubular treatment device in a radially contracted state to the affected area, and allows the tubular treatment device to radially expand in the affected area, whereby the tubular treatment device is placed in the affected area. A shaft-like member (inner tube) that has held the tubular treatment device during the placement is pulled out and removed from an inside of the tubular treatment device after the placement.

### Prior Art Document

### Patent Document

[Patent Document 1] JP-T-2008-504899
[Patent Document 2] JP-T-2016-511042

### Summary of Invention

### Technical Problem

In a case where a tubular treatment device is placed in a bent site of a biological lumen by using this type of indwelling device, there is a concern that a step formed in the shaft-like member catches on the tubular treatment device during the removal of the indwelling device and the shaft-like member cannot be smoothly pulled out.

Therefore, the present invention has been made in view of the above-described circumstances, and an object of the present invention is to provide an indwelling device capable of suppressing a step of a shaft-like member from being caught on a tubular treatment device in a case of placing the tubular treatment device in a bent site of a biological lumen, and allowing the shaft-like member to be smoothly pulled out.

### Solution to Problem

According to an aspect of the present invention, there is provided an indwelling device for placing a tubular treatment device, which is radially expandable, in a bent site in a biological lumen. The indwelling device includes: a sheath configured to accommodate the tubular treatment device; and a long shaft-like member configured to hold the tubular treatment device and to cause a distal side to advance and retreat inside the sheath along an axial direction. The shaft-like member includes a step portion that protrudes in a radial direction of the shaft-like member, and a covering portion that covers a boundary portion between the step portion and the shaft-like member with an inclined surface on a proximal side of the step portion.

### Advantageous Effects of Invention

According to the present invention, it is possible to provide an indwelling device capable of suppressing a step of a shaft-like member from being caught on a tubular treatment device in a case of placing the tubular treatment device in a bent site of a biological lumen, and allowing the shaft-like member to be smoothly pulled out.

### Brief Description of Drawings

Fig. 1(a) is a view showing a configuration example of a stent graft of the present embodiment, and Fig. 1(b) is a cross-sectional view taken along line Ib-Ib of Fig. 1(a).
Fig. 2(a) is a view showing a placement state of the stent graft of the present embodiment, and Fig. 2(b) is an enlarged view of a hepatic portal region of Fig. 2(a).
Fig. 3(a) is an exploded view of an indwelling device of the present embodiment, Fig. 3(b) is a view showing an assembled state of the indwelling device 1 of the present embodiment, and Fig. 3(c) is a view showing a state in which an inner tube is inserted into the stent graft.
Fig. 4 is an enlarged view of one side of the inner tube.
Fig. 5 is a view showing a state in which the stent graft is attached in Fig. 4.
Fig. 6 is a cross-sectional view taken along line VI-VI of Fig. 5.
Fig. 7 is a view showing a procedure of placing the stent graft using the indwelling device.
Fig. 8 is a view showing the procedure of placing the stent graft using the indwelling device.
Fig. 9 is a view schematically showing a state when the indwelling device is removed from an inside of the stent graft.

### Description of Embodiments

Hereinafter, configuration examples of an indwelling device and a tubular treatment device according to an embodiment of the present invention will be described with reference to the drawings.

A shape or a dimension of each portion in the drawings is schematically illustrated, and does not indicate an actual shape or an actual dimension. In the drawings, an axial direction Ax of the indwelling device and the tubular treatment device are indicated by arrows as necessary. In addition, a direction substantially orthogonal to the axial direction Ax is defined as a radial direction. As necessary, one side (distal side) of the indwelling device and the tubular treatment device in the drawings is indicated by reference numeral F, and the other side (proximal side) is indicated by reference numeral B.

First, a configuration of a stent graft 10 of the present embodiment will be described.

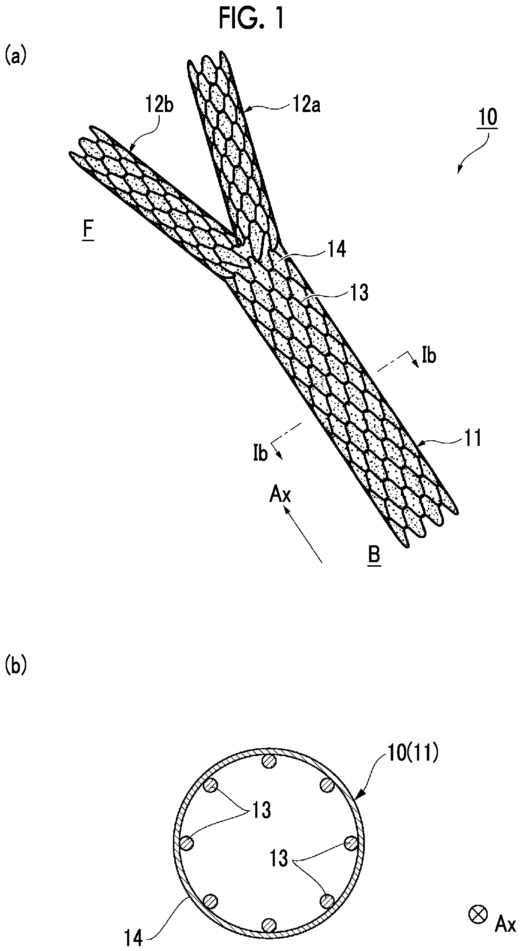

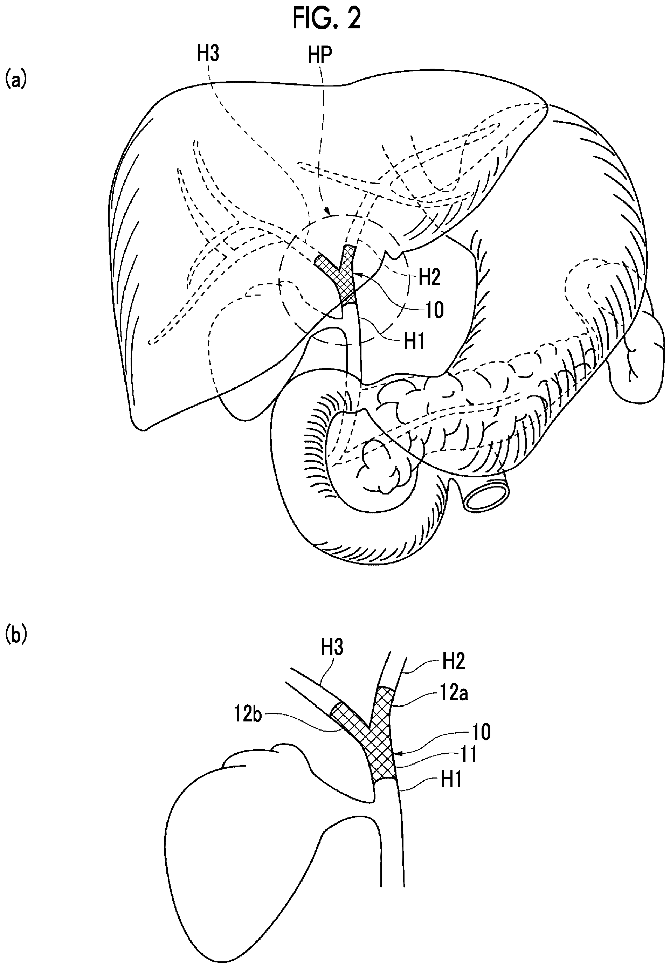

Fig. 1(a) is a view showing a configuration example of the stent graft 10 of the present embodiment, and Fig. 1(b) is a cross-sectional view taken along line Ib-Ib of Fig. 1(a). Fig. 2(a) is a view showing a placement state of the stent graft 10 of the present embodiment, and Fig. 2(b) is an enlarged view of a hepatic portal region HP of Fig. 2 (a).

The stent graft 10 is an example of the tubular treatment device, and is applied to expand a lesion site such as a narrowed site or an occluded site in a biological lumen by being placed in the lesion site using an indwelling device 1, which will be described below. As shown in Figs. 2(a) and 2(b), the stent graft 10 of the present embodiment is a covered stent for a bile duct and is placed in a hepatic portal region, which is an example of a biological lumen.

The stent graft 10 includes a first main body portion 11 and second main body portions 12a and 12b that branch from one side of the first main body portion 11. As shown in Figs. 2(a) and 2(b), the first main body portion 11 is placed in a common hepatic duct H1, and the second main body portions 12a and 12b are placed in a right hepatic duct H2 and a left hepatic duct H3, respectively.

Each of the first main body portion 11 and the second main body portions 12a and 12b is formed in a tubular shape. The second main body portions 12a and 12b have a smaller tube diameter than the first main body portion 11 and are connected to an end portion of one side of the first main body portion 11 so as to branch from the end portion into two portions. Accordingly, one side of the first main body portion 11 communicates with the other side of each of the second main body portions 12a and 12b, and the first main body portion 11 and the second main body portions 12a and 12b form a Y-shaped bile flow passage therein. In addition, an angle of a crotch portion where the second main body portions 12a and 12b branch is determined according to a shape of the hepatic portal region HP in which the stent graft 10 is to be placed.

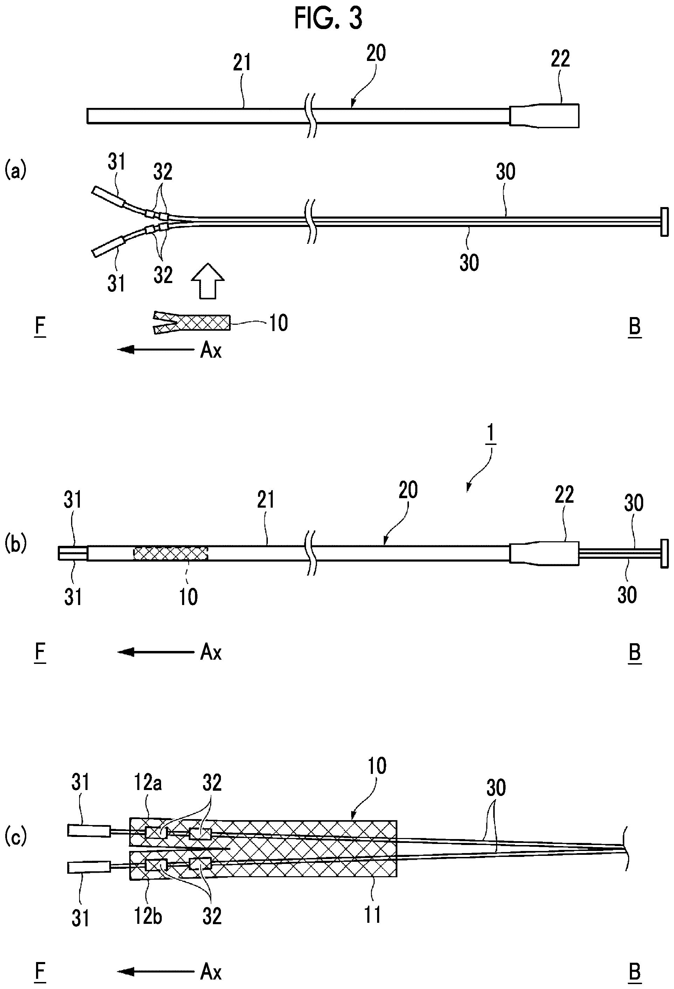

In addition, as shown in Fig. 3(c), when the second main body portions 12a and 12b are aligned and arranged along the axial direction, the second main body portions 12a and 12b are disposed in a state in which respective axial centers thereof are offset from an axial center of the first main body portion 11. Accordingly, the second main body portions 12a and 12b are easily compressed in the radial direction, and the stent graft 10 is easily accommodated in a sheath 20 of the indwelling device 1, which will be described below. In the following description, in a case where matters common to the second main body portions 12a and 12b are described, the second main body portions 12a and 12b may be collectively referred to as a second main body portion 12.

The first main body portion 11 and the second main body portions 12a and 12b each have a skeleton portion 13 and a membrane portion 14 fixed to the skeleton portion 13.

The skeleton portion 13 has a so-called self-expanding configuration in which a shape in an expanded state is memorized, and can expand and contract from a contracted state of being contracted radially inward to an expanded state of being expanded radially outward. Therefore, the stent graft 10 in the placement state presses inner surfaces of the common hepatic duct H1, the right hepatic duct H2, and the left hepatic duct H3 by a self-expanding force of the skeleton portion 13. In addition, in the stent graft 10 in the placement state, the skeleton portion 13 disposed in each of the first main body portion 11 and the second main body portions 12a and 12b can be deformed in response to an external force applied from an outside.

The skeleton portion 13 is configured in a tubular shape formed by weaving a wire material consisting of metal wires in a fence shape, for example. Examples of a material of the wire material of the skeleton portion 13 include known metals or metal alloys typified by a Ni-Ti alloy, stainless steel, and a titanium alloy. The skeleton portion 13 may be formed of a material other than metal (for example, ceramic or a resin).

In addition, an alloy material having X-ray visibility may be used for the wire material of the skeleton portion 13, or a marker piece (not shown) formed of an alloy material having X-ray visibility may be attached to the wire material as appropriate. In these cases, a position of the stent graft 10 can be checked from outside the body.

In a case where the Ni-Ti alloy is used as the material forming the skeleton portion 13, the skeleton portion 13 can be made to memorize the shape of the expanded state by adjusting the skeleton portion 13 to the shape of the expanded state and then performing a predetermined heat treatment thereon.

It should be noted that the configuration of the skeleton portion 13 is not limited to the above. For example, the skeleton portion 13 may be formed by spirally winding a thin metal wire bent in a zigzag shape. Alternatively, the skeleton portion 13 may have a structure in which a plurality of ring-shaped skeleton pieces in which thin metal wires folded in a zigzag shape are connected in an annular shape are arranged at intervals in the axial direction. In addition, the skeleton portion 13 may also be formed by performing laser cutting on a thin cylindrical body made of various metals mentioned above.

The membrane portion 14 is a tubular flexible membrane body that forms the above-described flow passage, and is attached to the skeleton portion 13 to close gap portions of the skeleton portion 13. The membrane portion 14 is attached to an outer peripheral surface of the skeleton portion 13, for example, as shown in Fig. 1(b). Accordingly, the membrane portion 14 suppresses an event (ingrowth) of infiltration of a cell tissue of the lesion site into the skeleton portion 13. The membrane portion 14 may be attached to an inner peripheral surface of the skeleton portion 13, or may be attached to the outer peripheral surface and the inner peripheral surface of the skeleton portion 13 so as to sandwich the skeleton portion 13.

Examples of a material that forms the membrane portion 14 include a silicone resin, a fluororesin such as polytetrafluoroethylene (PTFE), and a polyester resin such as polyethylene terephthalate.

In addition, examples of a method of fixing the membrane portion 14 to the skeleton portion 13 include formation of a membrane by dipping. However, the method of fixing the membrane portion 14 may also be sewing with a thread, adhesion, welding, bonding with a tape, or the like.

It should be noted that a removal assist portion (lasso) (not shown) may be connected to an end portion of the other side of the first main body portion 11 of the stent graft 10. The removal assist portion includes an engagement portion with which a hooking device provided at a distal end of a recovery catheter is engaged, and is used to remove the stent graft 10 that is placed in the hepatic portal region HP. The above-described engagement portion is formed, for example, by bending a wire material. The engagement portion may have, for example, a hook shape or a loop shape.

Next, the configuration example of the indwelling device in the present embodiment will be described.

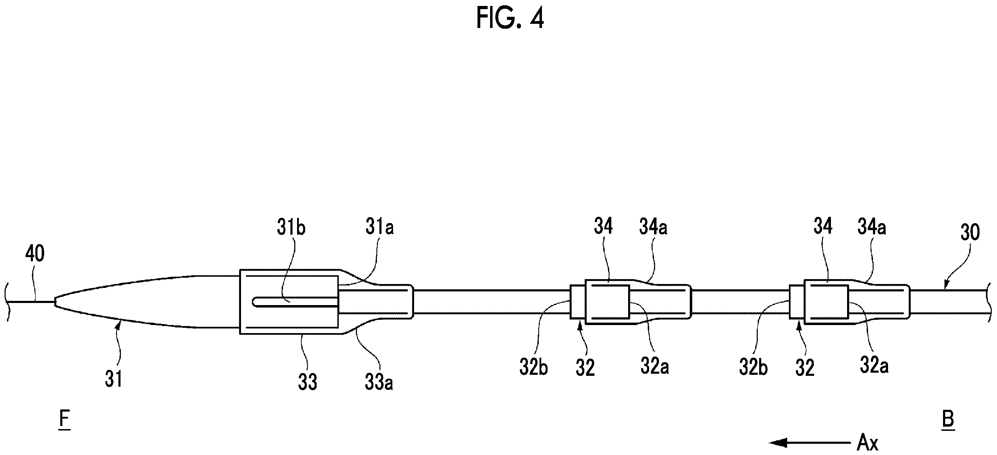

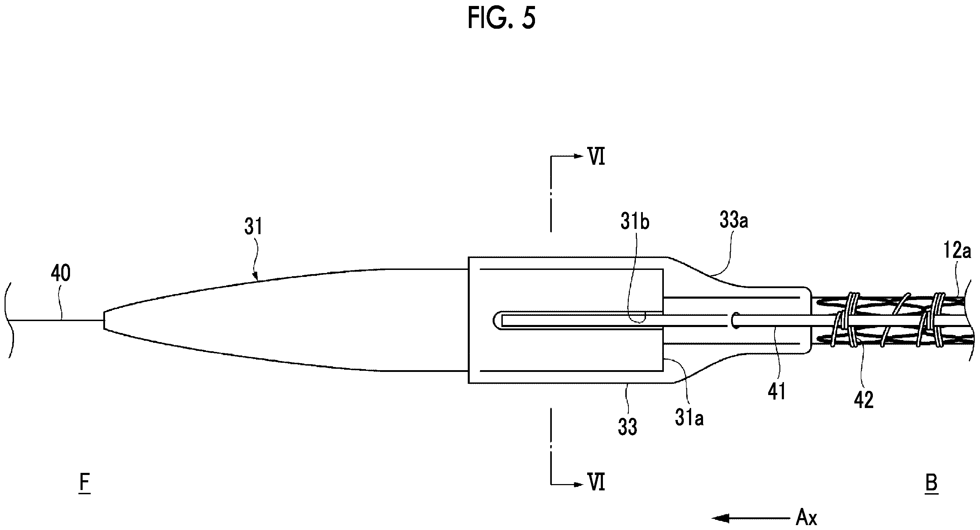

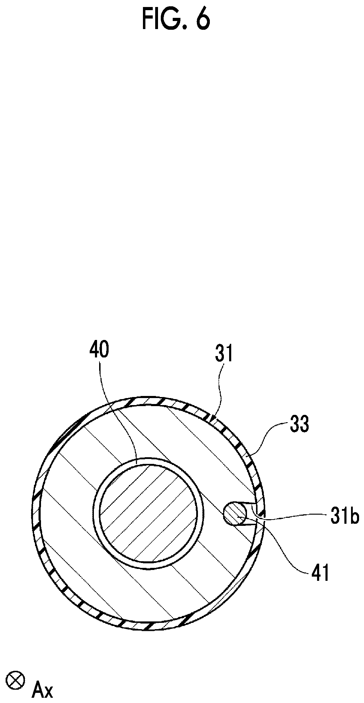

Fig. 3(a) is an exploded view of the indwelling device 1 of the present embodiment, and Fig. 3(b) is a view showing an assembled state of the indwelling device 1 of the present embodiment. Fig. 3(c) is a view showing a state in which an inner tube 30 is inserted into the stent graft 10. Fig. 4 is an enlarged view of one side of the inner tube 30. Fig. 5 is a view showing a state in which the stent graft 10 is attached in Fig. 4. Fig. 6 is a cross-sectional view taken along line VI-VI of Fig. 5.

As shown in Fig. 3(a), the indwelling device 1 includes the tubular sheath 20 and the tubular inner tube 30 disposed inside the sheath 20.

The sheath 20 can accommodate the stent graft 10 in a contracted state therein. The sheath 20 includes a sheath main body portion 21 and a hub 22 provided in an end portion of the other side of the sheath main body portion 21. The hub 22 includes a nut (not shown) for fixing the sheath 20 to the inner tube 30, an operating member (not shown) for operating a deployment wire 41, which will be described below, and the like.

The sheath main body portion 21 is a tubular body formed of a flexible material. Examples of a material of the sheath main body portion 21 include a biocompatible synthetic resin (elastomer) selected from a fluororesin, a polyamide resin, a polyethylene resin, a polyvinyl chloride resin, and the like, a resin compound in which other materials are mixed with these resins, a multilayer structure formed of the synthetic resins, and a composite of the synthetic resins and metal wires.

The inner tube 30 is a shaft-like member that is longer has a smaller diameter than sheath 20. The inner tube 30 is disposed inside the sheath 20 and is configured to cause a distal side to advance and retreat in the axial direction Ax with respect to the sheath 20.

As shown in Fig. 3(c), a pair of the inner tubes 30 are disposed in the sheath 20 in order to be inserted through the second main body portion 12a to be placed in the right hepatic duct H2 and through the second main body portion 12b to be placed in the left hepatic duct H3, respectively. Since configurations of the pair of inner tubes 30 are the same, the inner tube 30 corresponding to the second main body portion 12a will be described in the following description, and redundant descriptions of the inner tube 30 corresponding to the second main body portion 12b will be omitted.

The inner tube 30 is a tubular body having a hole communicating from one side to the other side in the axial direction Ax. A guide wire 40 which is to be disposed in the hepatic portal region HP, which will be described below, is inserted through the hole of the inner tube 30. Examples of a material of the inner tube 30 include various materials having appropriate hardness and flexibility, such as a resin (plastic, elastomer, and the like) and a metal.

On one side of the indwelling device 1, the stent graft 10 is accommodated in a space formed between an outer periphery of the inner tube 30 and an inner periphery of the sheath main body portion 21. As shown in Fig. 3(c), the inner tube 30 corresponding to the second main body portion 12a is inserted through the first main body portion 11 and the second main body portion 12a of the stent graft 10 and is disposed in the sheath 20. Similarly, the inner tube 30 corresponding to the second main body portion 12b is inserted through the first main body portion 11 and the second main body portion 12b of the stent graft 10 and is disposed in the sheath 20.

In a case where the stent graft 10 is attached to the inner tube 30, as shown in Fig. 5, the second main body portion 12a through which the inner tube 30 is inserted is held in the inner tube 30 in a state in which the second main body portion 12a is restrained from the outside by a string-like holding member 42 wound around an outer periphery of the second main body portion 12a and the deployment wire 41 engaged with the holding member 42 and is thus contracted.

The deployment wire 41 is an example of a linear member, and is disposed in the sheath 20 to extend in the axial direction in parallel with the inner tube 30. The other side of the deployment wire 41 is connected to the operating member of the hub 22 through the inside of the sheath 20. The deployment wire 41 can be pulled out along the axial direction by operating the operating member.

The holding member 42 is wound around an outer peripheral surface of the second main body portion 12a in such a manner that the holding member 42 cannot hold a wound state by itself, and is held so as not to fall off by being engaged with the deployment wire 41. As an example, the holding member 42 is hooked onto the deployment wire 41, folded back, and wound around the second main body portion 12a. At this time, by appropriately pulling both ends of the holding member 42 to apply tension, the second main body portion 12a of the stent graft 10 is reduced in diameter.

On the other hand, in a case where the engagement between the holding member 42 and the deployment wire 41 is released by pulling out the deployment wire 41, the holding member 42 falls off from the outer periphery of the second main body portion 12a. Accordingly, restriction on radial expansion of the second main body portion 12a can be released.

Here, the deployment wire 41 is formed of a material having a predetermined strength and rigidity, and examples of the material include a nickel-titanium alloy and a thin metal wire made of stainless steel. In addition, as a material of the holding member 42, for example, a suture such as nylon fibers and fluorine fibers, and a string-like member made of a resin can be applied.

In addition, as shown in Fig. 4, a distal tip 31 and a rib 32 are attached to one side of the inner tube 30.

The distal tip 31 is a tubular member attached to one side (distal side) of the inner tube 30. The distal tip 31 has a function of suppressing the inner tube 30 from coming into contact with a biological tissue of a patient in a case where the indwelling device 1 is inserted into the body to place the stent graft 10. In addition, examples of a material of the distal tip 31 include various materials having appropriate hardness and flexibility, such as a resin (plastic, elastomer, and the like) and a metal.

The distal tip 31 has a larger diameter than the inner tube 30, and a shape of one side of the distal tip 31 has a taper shape that gradually tapers toward one side. On the other side (proximal side) of the distal tip 31, an annular step portion 31a that protrudes radially from the inner tube 30 is formed. In addition, a hole for allowing the guide wire 40 to be inserted therethrough is formed at a center of the distal tip 31 along the axial direction.

In addition, a recessed portion 31b that receives a distal portion of one side of the deployment wire 41 is formed on a part of an outer periphery of the distal tip 31. The recessed portion 31b is recessed inward from an outer peripheral surface of the distal tip 31 and has a groove shape extending in the axial direction from the proximal side toward the distal side. A specification (width in a circumferential direction and depth in the radial direction) of the recessed portion 31b is determined within a range in which the distal portion of the deployment wire 41 can be accommodated in the recessed portion 31b.

In addition, a covering portion 33 that covers the step portion 31a and the recessed portion 31b of the distal tip 31 from the outside with a thin film member is formed on the proximal side of the distal tip 31. The covering portion 33 of the distal tip 31 has a function of causing the step portion 31a of the proximal side of the distal tip 31 to be less likely to catch on the skeleton portion 13 and a function of retaining the deployment wire 41 in the recessed portion 31b of the distal tip 31.

The covering portion 33 is formed in the axial direction from a distal end of the recessed portion of the distal tip 31 to the inner tube 30, with one side attached to an outer periphery of the distal tip 31 and the other side attached to the outer periphery of the inner tube 30. As an example, the covering portion 33 forms a tapered inclined surface 33a from the proximal side of the distal tip 31 to the inner tube 30, and the proximal side of the distal tip 31 and the inner tube 30 are connected to each other by the tapered inclined surface 33a in the axial direction. The tapered inclined surface 33a may be formed by attaching the covering portion 33, or may be formed by performing processing after the covering portion 33 is attached. In addition, when the inner tube 30 is pulled out after the stent graft 10 is placed, the covering portion 33 located on the proximal side advances within the stent graft 10 ahead of the distal tip 31.

In addition, as shown in Fig. 6, the covering portion 33 attached to the distal tip 31 covers the outer periphery of the proximal side of the distal tip 31. Accordingly, the recessed portion 31b of the distal tip 31 is blocked by the thin film member of the covering portion 33 at a position of the outer peripheral surface of the distal tip 31, and a distal end of the deployment wire 41 accommodated in the recessed portion 31b is suppressed from protruding from the recessed portion 31b toward an outer peripheral side.

In addition, the covering portion 33 tightens the proximal side of the distal tip 31 from the outside in the circumferential direction. Accordingly, a width of the recessed portion 31b that accommodates the deployment wire 41 is narrowed in the circumferential direction, so that the distal end of the deployment wire 41 is sandwiched and held in the recessed portion 31b. Therefore, even with the tightening of the distal tip 31 by the covering portion 33, the distal end of the deployment wire 41 is less likely to protrude from the recessed portion 31b to the outer peripheral side.

As a material of the covering portion 33, a known thin film member having elasticity and biocompatibility is used, and the covering portion 33 is formed of, for example, a material having higher elasticity than the material of the distal tip 31. In a case where the elasticity of the covering portion 33 is higher than that of the distal tip 31, even in a case where contact occurs between the covering portion 33 and the skeleton portion 13 when the inner tube 30 is pulled out, a force is distributed by the elasticity of the covering portion 33, and a positional shift of the skeleton portion 13 or the like is less likely to occur. A thickness of the covering portion 33 can be appropriately adjusted according to a filling rate in the sheath 20.

A thin film material of the covering portion 33 is not particularly limited, but preferably has a light transmitting property that allows transmission of visible light so that a state of attachment of the deployment wire 41 to the recessed portion 31b can be visually checked from the outside.

In addition, for example, the covering portion 33 may be attached to a formation site by heat-shrinking a tubular thin film material, or may be attached to a formation site by winding a sheet-like thin film material around the formation site. In a case where the covering portion 33 is formed by heat-shrinking the thin film material, blocking of the recessed portion 31b can be suppressed by disposing a dummy wire material or the like in the recessed portion 31b as necessary during the processing.

The rib 32 is a protruding body formed in a region where the stent graft 10 is held on one side of the inner tube 30. The rib 32 has a function of transmitting a thrust toward the one side to the stent graft 10 held by the inner tube 30 when the inner tube 30 is caused to advance to one side with respect to the sheath 20.

The rib 32 has a cylindrical shape having a larger diameter than the inner tube 30, and is attached to the inner tube 30 at an interval from the distal tip 31 to the other side. Figs. 3 and 4 show an example in which two ribs 32 are disposed in regions where the stent graft 10 is held, but the number of ribs 32 provided in the inner tube 30 is not limited to the above.

Each of the ribs 32 has annular step portions 32a and 32b that protrude radially from the inner tube 30 on one side and the other side, respectively. The step portion 32b of one side of the rib 32 is an example of a first step, and is exposed to the outside in order to transmit a thrust toward one side to the stent graft 10. On the other hand, on a proximal side of the rib 32, a covering portion 34 that covers the step portion 32a of the proximal side of the rib 32 from the outside with a thin film member is formed. The covering portion 34 of the rib 32 has a function of causing the step portion 32a of the proximal side of the rib 32 to be less likely to catch on the skeleton portion 13. The step portion 32a is an example of a second step.

The covering portion 34 is formed in the axial direction from the proximal side of the rib 32 to the inner tube 30, with one side attached to an outer periphery of the rib 32 and the other side attached to the outer periphery of the inner tube 30. As an example, the covering portion 34 forms a tapered inclined surface 34a from the proximal side of the rib 32 to the inner tube 30, and the proximal side of the rib 32 and the inner tube 30 are connected to each other by the tapered inclined surface 34a in the axial direction. The tapered inclined surface 34a may be formed by attaching the covering portion 34, or may be formed by performing processing after the covering portion 34 is attached. In addition, when the inner tube 30 is pulled out after the stent graft 10 is placed, the covering portion 34 located on the proximal side advances within the stent graft 10 ahead of the rib 32.

A material of the covering portion 34 attached to the rib 32 and an attachment method thereof are the same as those of the covering portion 33 of the distal tip 31, and redundant descriptions thereof will be omitted.

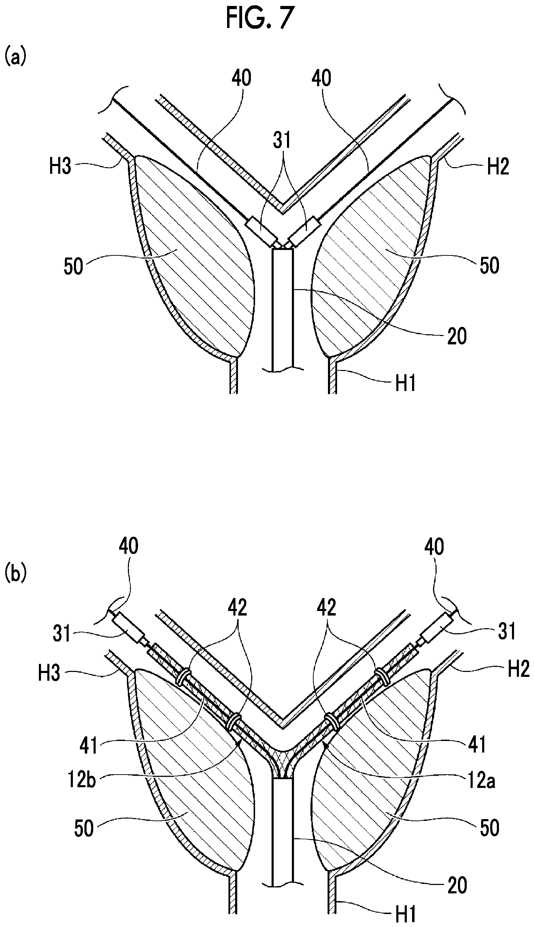

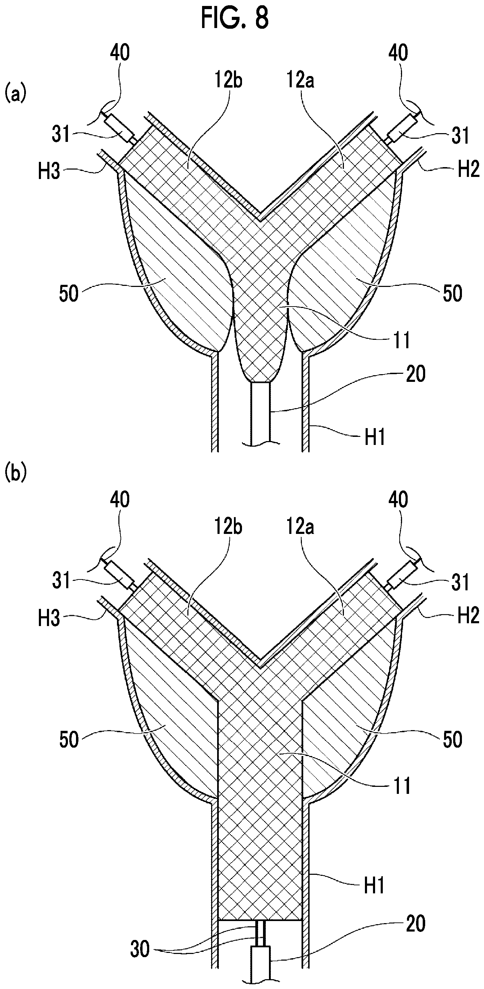

Next, a procedure of placing the stent graft 10 in the hepatic portal region using the indwelling device 1 will be described with reference to Figs. 7 and 8.

First, the guide wires 40 are respectively disposed in the right hepatic duct H2 and the left hepatic duct H3 branching from the common hepatic duct H1 so that the guide wires 40 pass through a lesion site 50 in which an aneurysm is formed. Then, regarding the indwelling device 1 with the stent graft 10 in the contracted state accommodated in the sheath 20, the guide wires 40 are inserted into the indwelling device 1 from an end portion of one side of the indwelling device 1. Thereafter, the indwelling device 1 is introduced into the patient's body, and the indwelling device 1 with the stent graft 10 in the contracted state accommodated in the sheath 20 is caused to advance to a position where the common hepatic duct H1 branches, as shown in Fig. 7(a).

Next, as shown in Fig. 7(b), the pair of inner tubes 30 and 30 that hold the second main body portions 12a and 12b in the contracted state are caused to advance to one side (the distal side) in the right hepatic duct H2 and the left hepatic duct H3, respectively, while the sheath 20 is retained at the position where the common hepatic duct H1 branches. At this time, the thrust toward one side to the inner tubes 30 and 30 is transmitted to the second main body portion 12 via the step portion 32b of one side of the rib 32, and the second main body portion 12 advances to one side in synchronization with a movement of the inner tubes 30 and 30.

In addition, by causing each inner tube 30 to advance to one side, the deployment wire 41 is also exposed outside the sheath 20 on one side of the indwelling device 1. However, since the distal portion of the deployment wire 41 is retained in the recessed portion 31b of the distal tip 31, the distal portion of the deployment wire 41 does not come into contact with the patient's body. Therefore, invasion caused by the distal portion of the deployment wire 41 coming into contact with the biological lumen is suppressed.

Next, when each of the inner tubes 30 advances to a predetermined position, the deployment wire 41 is pulled out to the other side on each of a right hepatic duct H2 side and a left hepatic duct H3 side. Then, the engagement between the holding member 42 and the deployment wire 41 is released, and the holding member 42 falls off from the outer periphery of the second main body portion 12, so that the restriction on the radial expansion of the second main body portion 12 is released. Accordingly, as shown in Fig. 8(a), the second main body portions 12a and 12b self-expand radially outward on the right hepatic duct H2 side and the left hepatic duct H3 side, respectively.

Thereafter, the sheath 20 is moved to be pulled out toward the other side. Then, as shown in Fig. 8(b), the first main body portion 11 of the stent graft 10 is released from the sheath 20 of the indwelling device 1. The first main body portion 11 of the stent graft 10 self-expands radially outward as the first main body portion 11 is released to the outside from the sheath 20. Accordingly, the stent graft 10 is placed in the hepatic portal region HP, and the lesion site 50 is expanded.

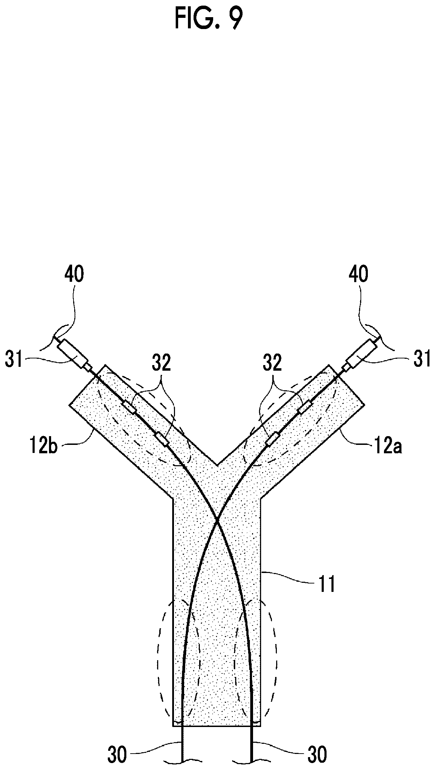

After the placement of the stent graft 10 is completed, the indwelling device 1 and the guide wire 40 are moved to the other side and removed from the body. Fig. 9 is a view schematically showing a state when the indwelling device 1 is removed from an inside of the stent graft 10.

In a state in which the stent graft 10 is deployed, the inner tube 30 is inserted through the first main body portion 11 and the second main body portion 12, and the distal tip 31 advances to one side beyond the second main body portion 12. Therefore, when the inner tube 30 is pulled out to the other side, the distal tip 31 and the rib 32 attached to the inner tube 30 pass through insides of the second main body portion 12 and the first main body portion 11 in order.

In addition, the guide wire 40 is inserted through the inner tube 30. The guide wire 40 has predetermined linearity for stable advance of the indwelling device 1 in the biological lumen, but the guide wire 40 is disposed to be bent at the hepatic portal region HP that branches to the left and right. Therefore, the two guide wires 40 at the hepatic portal region HP undergo a restoring force that tries to return to a straight line shape when pulled out, and thus take trajectories with a largest possible radius of curvature.

As a result, the guide wires 40 approach an inner wall of the stent graft 10. The inner tubes 30 through which the guide wires 40 are inserted also follow the trajectories of the guide wires 40, so that the distal tip 31 and the rib 32 protruding radially from the inner tube 30 pass through the vicinity of the inner wall of the stent graft 10, for example, at portions indicated by broken lines in Fig. 9 when pulled out.

The distal tip 31 and the rib 32 in the present embodiment respectively have the covering portion 33 and 34 that cover boundary portions between the step portions 31a and 32a on the other side (proximal side) and the inner tube 30 with the tapered inclined surface 33a and 34a. When the inner tube 30 is pulled out to the other side, the inclined surface 34a of the covering portion 34 formed on the other side of the rib 32 advances within the stent graft 10 toward the other side ahead of the rib 32. Similarly, when the inner tube 30 is pulled out to the other side, the inclined surface 33a of the covering portion 33 formed on the other side of the distal tip 31 advances within the stent graft 10 toward the other side ahead of the distal tip 31.

The inclined surfaces 33a and 34a of the covering portion 33 and 24 gradually change in diameter from the outer periphery of the inner tube 30 to the rib 32 or the distal tip 31 in the axial direction. That is, on the other side of the rib 32 or the distal tip 31, a change in the radial direction (step) is reduced by the covering portions 33 and 34. When the inner tube 30 is pulled out to the other side, an object in contact with the inner tube 30 slides on the inclined surfaces 33a and 34a of the covering portions 33 and 34, rides on the step portion 31a of the proximal side of the distal tip 31 or the step portion 32a of the proximal side of the rib 32, and is guided to the outer periphery of the distal tip 31 or the rib 32. Therefore, when the inner tube 30 is pulled out to the other side, the skeleton portion 13 of the stent graft 10 is suppressed from being caught on the distal tip 31 or on the rib 32.

Hereinafter, an advantageous effect of the indwelling device 1 of the present embodiment will be described.

In the present embodiment, the indwelling device 1 for placing the stent graft 10 (tubular treatment device), which is radially expandable, in the hepatic portal region HP (a bent site in a biological lumen) includes: the sheath 20 configured to accommodate the stent graft 10; and the long inner tube 30 (shaft-like member) configured to hold the stent graft 10 and to cause one side (distal side) to advance and retreat inside the sheath 20 along the axial direction. The inner tube 30 includes members (the distal tip 31 and the rib 32) that form the step portions 31a and 32a protruding in the radial direction of the inner tube 30, and the covering portions 33 and 34 that cover the boundary portions between the step portions 31a and 32a and the inner tube 30 with the inclined surfaces 33a and 34a on the other side (proximal side) of the step portions 31a and 32a.

In the indwelling device 1 of the present embodiment, an object in contact with the inner tube 30 rides on the step portions 31a and 32a due to the inclined surfaces 33a and 34a of the covering portions 33 and 34. Therefore, the lasso connected to the skeleton portion 13 or the first main body portion 11 of the stent graft 10 is suppressed from being caught on the step portions 31a and 32a, and migration or the like of the stent graft 10 that has been placed is less likely to occur.

In addition, the covering portions 33 and 34 also suppress the step portions 31a and 32a of the inner tube 30 pulled out from the stent graft 10 from coming into contact with an inner wall of the biological lumen.

In addition, the guide wire 40 disposed in the biological lumen is inserted through the inner tube 30 in the axial direction. Due to an action of the linearity of the guide wire 40, the step portions 31a and 32a approach the inner wall of the stent graft 10 when the inner tube 30 is pulled out. However, the covering portions 33 and 34 can suppress the skeleton portion 13 from being caught on the step portions 31a and 32a.

In addition, the covering portions 33 and 34 are formed of a thin film member attached to the step portions 31a and 32a. By forming the covering portions 33 and 34 of the thin film member, the inclined surfaces 33a and 34a that cover the step portions 31a and 32a can be formed by relatively simple processing.

In addition, in a case where the covering portions 33 and 34 have higher elasticity than the member forming the step portions 31a and 32a, even in a case where contact occurs between the covering portions 33 and 34 and the skeleton portion 13 occurs when the inner tube 30 is pulled out, a force is distributed by the elasticity of the covering portions 33 and 34, and a positional shift of the skeleton portion 13 or the like is less likely to occur.

In addition, in a case where a tubular treatment device is placed in a biological lumen by using this type of indwelling device, there is a concern that a distal end of a linear member, which is disposed to control deployment of the tubular treatment device, comes into contact with an inner wall of the biological lumen, causing an invasion of the patient's body.

Contrary to this, one aspect of the present embodiment can provide a indwelling device capable of suppressing the distal end of the linear member from coming into contact with the inner wall of the biological lumen in a case where the tubular treatment device is placed in the biological lumen.

In the present embodiment, the indwelling device 1 for placing the stent graft 10 (tubular treatment device), which is radially expandable, in the hepatic portal region HP (biological lumen) includes: the sheath 20 configured to accommodate the stent graft 10; the long inner tube 30 (shaft-like member) configured to cause one side (distal side) to advance and retreat inside the sheath 20 along the axial direction; the deployment wire 41 (linear member) that extends along the inner tube 30 in the axial direction to hold the stent graft 10 in a contracted state in the inner tube 30; and the distal tip 31 that is disposed at a distal portion of the inner tube 30. The distal tip 31 includes the recessed portion 31b that is recessed from a surface of the distal tip and that receives an end portion of the deployment wire 41.

In the indwelling device 1 of the present embodiment, the end portion of the deployment wire 41 is accommodated in the recessed portion 31b of the distal tip 31, so that the distal portion of the deployment wire 41 exposed outside the sheath 20 does not come into contact with the patient's body. Therefore, it is possible to suppress the invasion caused by the distal portion of the deployment wire 41 being pierced into the biological lumen.

In addition, the recessed portion 31b has a groove shape extending from the other side (proximal side) of the distal tip 31 in the axial direction, and the distal tip 31 further includes the covering portion 33 that covers the recessed portion 31b from the outer peripheral side and that retains the end portion of the deployment wire 41 inside the recessed portion 31b. Therefore, the recessed portion 31b is blocked by the covering portion 33 at a position of the outer peripheral surface of the distal tip 31, and the distal end of the deployment wire 41 accommodated in the recessed portion 31b is suppressed from protruding from the recessed portion 31b toward the outer peripheral side.

In addition, the end portion of the deployment wire 41 is sandwiched and held in the recessed portion 31b tightened in the circumferential direction by the covering portion 33. Therefore, a holding force of the deployment wire 41 in the recessed portion 31b of the distal tip 31 is improved by the tightening of the covering portion 33, and the distal end of the deployment wire 41 is suppressed from moving from the recessed portion 31b toward the outer peripheral side.

In addition, in a case where the covering portion 33 is formed of a material having a light transmitting property, the state of attachment of the deployment wire 41 to the recessed portion 31b can be visually checked from the outside. Therefore, for example, the state of attachment of the deployment wire 41 can be checked without performing X-ray imaging or the like, thereby reducing efforts in quality checking work.

The present invention is not limited to the above-described embodiments, and various improvements and design changes may be made within the scope not departing from the concept of the present invention.

In the above-described embodiment, the configuration example of the indwelling device that places the stent graft to be disposed in the hepatic portal region has been described, but the indwelling device of the present invention is not limited to the above-described embodiment. For example, in the indwelling device that places the tubular treatment device in a biological lumen such as a blood vessel or a digestive tract having a bent site, the covering portion of the embodiment may be provided on the step portion of the shaft-like member.

Furthermore, for example, in general indwelling devices for placing a tubular treatment device in a biological lumen such as a blood vessel or a digestive tract, the recessed portion that receives the end portion of the deployment wire may be provided in the distal tip, as in the above-described embodiment.

In addition, in the above-described embodiment, the recessed portion may be formed on an inner peripheral side between the distal tip 31 and the inner tube 30, and the distal portion of the deployment wire 41 may be inserted into the recessed portion to be held. In this case, the distal portion of the deployment wire 41 can be held by the recessed portion without the covering portion 33.

In addition, the embodiments disclosed herein are merely examples in all respects, and should not be considered restrictive. The scope of the present invention is represented by the appended claims, not by the above description, and is intended to include all modifications within the meaning and the scope which are equivalent to those of the appended claims.

### Reference Signs List

1: indwelling device
10: stent graft
11: first main body portion
12a, 12b: second main body portion
13: skeleton portion
14: membrane portion
20: sheath
30: inner tube
31: distal tip
31a: step portion
31b: recessed portion
32: rib
32a, 32b: step portion
33, 34: covering portion
33a, 34a: inclined surface
40: guide wire

## Claims

1. An indwelling device for placing a tubular treatment device, which is radially expandable, in a bent site in a biological lumen, the indwelling device comprising:
a sheath configured to accommodate the tubular treatment device; and
a long shaft-like member configured to hold the tubular treatment device and to cause a distal side to advance and retreat inside the sheath along an axial direction,
wherein the shaft-like member includes
a step portion that protrudes in a radial direction of the shaft-like member, and
a covering portion that covers a boundary portion between the step portion and the shaft-like member with an inclined surface on a proximal side of the step portion.

2. The indwelling device according to claim 1,
wherein a wire that is to be disposed in the biological lumen is inserted through the shaft-like member in the axial direction.

3. The indwelling device according to claim 1,
wherein the step portion is a distal tip that is disposed at a distal portion of the shaft-like member.

4. The indwelling device according to claim 1,
wherein the step portion is a protruding body that is formed in a region of the shaft-like member where the tubular treatment device is held and that transmits a thrust toward the distal side to the tubular treatment device held by the shaft-like member.

5. The indwelling device according to claim 4,
wherein the protruding body has a first step that presses the tubular treatment device on the distal side and in which a boundary portion between a second step formed on the proximal side and the shaft-like member is covered with the covering portion.

6. The indwelling device according to claim 1,
wherein the covering portion is a thin film member attached to the step portion.

7. The indwelling device according to claim 1,
wherein the covering portion has higher elasticity than the step portion.

8. An indwelling device for placing a tubular treatment device, which is radially expandable, in a biological lumen, the indwelling device comprising:
a sheath configured to accommodate the tubular treatment device;
a long shaft-like member configured to cause a distal side to advance and retreat inside the sheath along an axial direction;
a linear member that extends along the shaft-like member in the axial direction to hold the tubular treatment device in a contracted state in the shaft-like member; and
a distal tip that is disposed at a distal portion of the shaft-like member,
wherein the distal tip includes a recessed portion that is recessed from a surface of the distal tip and that receives an end portion of the linear member.

9. The indwelling device according to claim 8,
wherein the recessed portion has a groove shape extending from a proximal side of the distal tip in the axial direction, and
the distal tip further includes a covering portion that covers the recessed portion from an outer peripheral side and that retains the end portion of the linear member inside the recessed portion.

10. The indwelling device according to claim 8,
wherein the end portion of the linear member is sandwiched and held in the recessed portion tightened in a circumferential direction by the covering portion.

11. The indwelling device according to claim 8,
wherein the covering portion is formed of a material having a light transmitting property.

12. The indwelling device according to any one of claims 1 to 11,
wherein the tubular treatment device includes a main body portion on a proximal side, and a first branch portion and a second branch portion that branch on a distal side of the main body portion, and
the indwelling device further comprises: a first shaft-like member inserted into the main body portion and the first branch portion; and a second shaft-like member inserted into the main body portion and the second branch portion.
